# EUROPEAN PATENT APPLICATION

(11) **EP 4 778 927 A1**
(43) Date of publication of application: **22.07.2026**
(21) Application number: 24864621.8
(22) Date of filing: 10.09.2024
(51) Int. Cl.: C07D 491/22, C07C 233/25, C07C 231/12

(54) **PREPARATION METHOD FOR CAMPTOTHECIN DERIVATIVE**

(30) Priority: 11.09.2023 CN 202311166462
(71) Applicant: Changzhou Syntheall Pharmaceuticals Co., Ltd., Changzhou, Jiangsu 213001 (CN)
(72) Inventor: LIU, Lang, hangzhou, Jiangsu 213001 (CN); YUAN, Changxia, hangzhou, Jiangsu 213001 (CN); JI, Tong, hangzhou, Jiangsu 213001 (CN); LIN, Hanfeng, hangzhou, Jiangsu 213001 (CN); SONG, Jifu, hangzhou, Jiangsu 213001 (CN)
(74) Representative: Cabinet Becker et Associés
(86) International application number: PCT/CN2024/118037
(87) International publication number: WO 2025/055905

(57) **Abstract**

Provided is a preparation method for a camptothecin derivative. Specifically provided is a preparation method for the compound of formula 7, comprising the following steps: in an organic solvent, performing a deprotection reaction on the compound of formula 6 and methanesulfonic acid, to obtain the compound of formula 7. The obtained preparation method uses a small amount of solvent during post-processing, and filtration thereof is easy. The prepared product is white, the product purity and yield are relatively high, and the method is suitable for industrial production. A recrystallization mother liquor obtained from the deprotection reaction is easier to recycle, and raw material costs are greatly reduced.

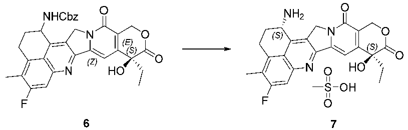

## Description

The present application claims the priority of Chinese patent application 2023111664626 filed on September 11, 2023. This Chinese patent application is incorporated herein by reference in its entirety.

### Technical Field

The present invention relates to a preparation method for a camptothecin derivative.

### Background Art

Exatecan is a novel camptothecin derivative that exhibits good water solubility and antitumor effects due to the introduction of an amino group. The structural formula of exatecan is as follows:

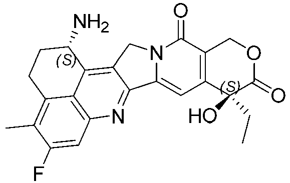

Exatecan is currently mainly synthesized using the classic route with acetyl as the amino protecting group, and this synthesis process is disclosed in patent CN111470998B. The specific synthesis route is as follows:

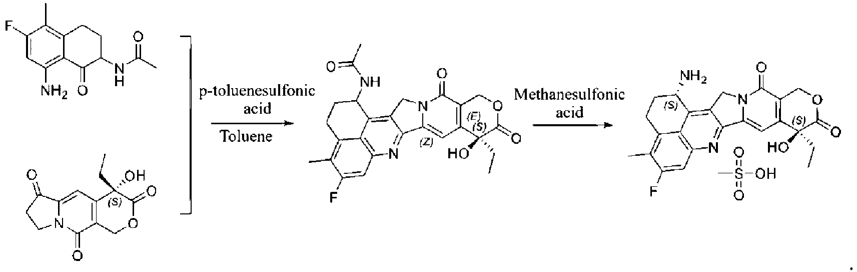

The synthesis process reported in patent CN111470998B was reproduced in the present invention; however, experimental results showed that a large amount of black substances were generated in the reaction solution, a high volume of solvent was required for crystallization, and the final yield was only 22.2% (on a corrected-content basis), with a purity of merely 95.4% and an isomer rate of 3.1%.

### Summary of the Invention

The technical problem to be solved by the present invention is that the existing preparation methods result in decomposition of the product during the reaction and use a large amount of crystallization solvent during the post-processing, resulting in long filtration time and low yield and purity. Therefore, the present invention provides a preparation method for a camptothecin derivative. The preparation method of the present invention uses a small amount of solvent in the post-processing and is easy to filter, and the prepared product is off-white, with higher purity and yield, and is suitable for industrial production. The recrystallization mother liquor obtained from the deprotection reaction is easier to recover, which greatly reduces the cost of raw materials.

The present invention provides a preparation method for a compound of formula 7, comprising the following steps: in an organic solvent, subjecting a compound of formula 6 to a deprotection reaction with methanesulfonic acid, to obtain the compound of formula 7,

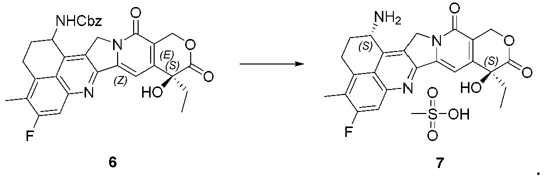

In one embodiment of the preparation method, certain technical features are defined as follows, and other technical features are defined as described in any of the following embodiments (hereinafter referred to as "in one embodiment").

In one embodiment, the preparation method for the compound of formula 7 can comprise the following specific steps: mixing the compound of formula 6 with the organic solvent, then mixing the mixture with the methanesulfonic acid for carrying out a deprotection reaction, to obtain the compound of formula 7.

In one embodiment, the mixing can be carried out at a temperature of 15-25°C.

In one embodiment, the molar ratio of the compound of formula 6 to methanesulfonic acid can be 1:(3-15), preferably 1:(3-10), e.g., 1:8.8.

In one embodiment, the volume-to-mass ratio of the compound of formula 6 to the organic solvent can be 1:(3-10) mL/g, preferably 1:(3-5) mL/g, e.g., 1:4.4 mL/g.

In one embodiment, the organic solvent can be a haloacetic acid, acetic acid or a chlorinated hydrocarbon solvent, the haloacetic acid is preferably trifluoroacetic acid, the chlorinated hydrocarbon solvent is preferably dichloromethane or chloroform, and the organic solvent is, e.g., trifluoroacetic acid.

In one embodiment, the deprotection reaction can be carried out at a temperature of 5-40°C, e.g., 15-25°C.

In one embodiment, a system of the deprotection reaction contains no water.

In one embodiment, the deprotection reaction can further comprise a post-processing step, which comprises one or more of crystallization, recrystallization, slurrying, filtration and drying.

In one embodiment, a solvent for the crystallization can be an alcoholic solvent and/or an ether solvent; the alcoholic solvent is preferably ethanol; the ether solvent is preferably tetrahydrofuran; the solvent for crystallization is preferably an alcoholic solvent and an ether solvent, e.g., ethanol and tetrahydrofuran.

In one embodiment, when the solvent for the crystallization is an alcoholic solvent and an ether solvent, the volume ratio of the alcoholic solvent to the ether solvent can be 1:(1-2), e.g., 1:1.5.

In one embodiment, the mass-to-volume ratio of the compound of formula 6 to the solvent for the crystallization can be 65.6 g/L.

In one embodiment, the solvent for the recrystallization can be a sulfoxide solvent and/or a nitrile solvent, preferably a sulfoxide solvent and a nitrile solvent. The sulfoxide solvent is preferably dimethyl sulfoxide; and the nitrile solvent is preferably acetonitrile.

In one embodiment, the volume ratio of the sulfoxide solvent to the nitrile solvent can be 1:3.

In one embodiment, a solvent for the slurrying can be a nitrile solvent, e.g., acetonitrile.

The preparation method for the compound of formula 7 can further comprise a preparation method for the compound of formula 6 which can comprise the following step: in a solvent and in the presence of a catalyst, carrying out a condensation reaction between a compound of formula 4 and a compound of formula 5, to obtain the compound of formula 6,

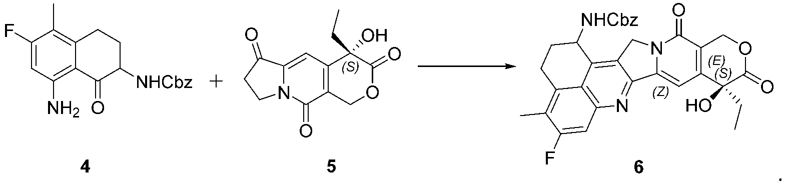

In one embodiment, the preparation method for the compound of formula 6 can comprise the following specific steps:
(a) mixing the compound of formula 4, the compound of formula 5, a portion of the catalyst and the solvent for carrying out a condensation reaction, to obtain reaction solution 1;
(b) mixing the remaining catalyst with the reaction solution 1 for carrying out a condensation reaction, to obtain the compound of formula 6.

In one embodiment, the solvent can be an aromatic solvent, e.g., toluene.

In one embodiment, the catalyst can be p-toluenesulfonic acid or pyridinium p-toluenesulfonate, e.g., p-toluenesulfonic acid.

In one embodiment, the molar ratio of the compound of formula 4 to the compound of formula 5 can be 1:(0.9-1.2), e.g., 1:1.05.

In one embodiment, the molar ratio of the compound of formula 4 to the catalyst can be 1:(0.1-0.3), e.g., 1:0.2.

In one embodiment, the mass ratio of the amount of the catalyst added in step (a) to that added in step (b) can be 1:1.

In one embodiment, the mass-to-volume ratio of the compound of formula 4 to the solvent is a conventional mass-to-volume ratio in the art, preferably 20 g/L-30 g/L, e.g., 20 g/L.

In one embodiment, the condensation reaction can be carried out at a reflux temperature of the solvent.

In one embodiment, the condensation reaction can further comprise a post-processing step, which comprises one or more of crystallization, slurrying, filtration and drying.

In one embodiment, a solvent for the slurrying can be an alcoholic solvent and/or a halogenated hydrocarbon solvent, preferably an alcoholic solvent and a halogenated hydrocarbon solvent. The alcoholic solvent is preferably methanol; and the halogenated hydrocarbon solvent is preferably dichloromethane.

The preparation method for the compound of formula 7 can further comprise a preparation method for the compound of formula 4 which can comprise the following step: in a solvent and in the presence of a catalyst, subjecting a compound of formula 3 to a substitution reaction with benzyl chloroformate, to obtain the compound of formula 4,

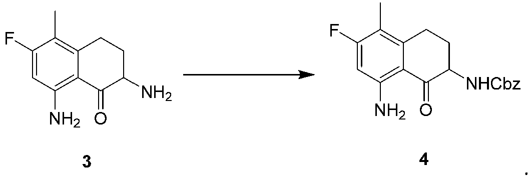

In one embodiment, the preparation method for the compound of formula 4 can comprise the following specific steps:
(a) mixing the compound of formula 3, the catalyst and the solvent, to obtain mixed solution 1;
(b) mixing the benzyl chloroformate and the mixed solution 1 for carrying out the substitution reaction, to obtain the compound of formula 4.

In one embodiment, the mixing in step (b) can be adding the benzyl chloroformate to the mixed solution 1.

In one embodiment, the solvent can be an ether solvent, preferably tetrahydrofuran.

In one embodiment, the catalyst can be N,N-diisopropylethylamine.

In one embodiment, the molar ratio of the compound of formula 3 to the catalyst can be 1:(3-4), e.g., 1:3.

In one embodiment, the molar ratio of the compound of formula 3 to the benzyl chloroformate can be 1:(1-1.5), e.g., 1:1.15.

In one embodiment, the mass-to-volume ratio of the compound of formula 3 to the solvent is a conventional mass-to-volume ratio in the art, preferably 5 g/L-6 g/L, e.g., 5.9 g/L.

In one embodiment, the substitution reaction can be carried out at a temperature of 10-20°C, e.g., 20°C.

In one embodiment, the substitution reaction can further comprise a post-processing step, which comprises one or more of extraction, washing, filtration, concentration, slurrying and drying.

The preparation method for the compound of formula 7 can further comprise a preparation method for the compound of formula 3 which can comprise the following step: in a solvent and in the presence of a catalyst and an acid, carrying out a catalytic hydrogenation reaction of a compound of formula 2 with H₂, to obtain the compound of formula 3,

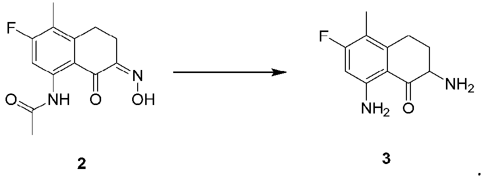

In one embodiment, the preparation method for the compound of formula 3 can comprise the following specific steps: mixing the compound of formula 2, the catalyst, the acid and the solvent for carrying out a catalytic hydrogenation reaction under an H₂ atmosphere, to obtain the compound of formula 3.

In one embodiment, the solvent can be an alcoholic solvent, e.g., methanol.

In one embodiment, the catalyst can be palladium on carbon (Pd/C), e.g., 10% Pd/C.

In one embodiment, the acid may be an organic acid, e.g., trifluoroacetic acid.

In one embodiment, the mass ratio of the compound of formula 2 to the catalyst can be 1:(0.002-0.003), e.g., 1:0.0025.

In one embodiment, the molar ratio of the compound of formula 2 to the acid can be 1:(2-4), e.g., 1:2.7.

In one embodiment, the mass-to-volume ratio of the compound of formula 2 to the solvent is a conventional mass-to-volume ratio in the art, preferably 90 g/L-110 g/L, e.g., 100 g/L.

In one embodiment, the catalytic hydrogenation reaction can be carried out at a temperature of 20-40°C, e.g., 25°C.

In one embodiment, the catalytic hydrogenation reaction can further comprise a post-processing step, which comprises one or more of dissolution, washing, filtration, concentration and drying.

The preparation method for the compound of formula 7 can further comprise a preparation method for the compound of formula 2 which can comprise the following step: in a solvent and in the presence of a base, subjecting a compound of formula 1 to an oximation reaction with an oximating agent, to obtain the compound of formula 2,

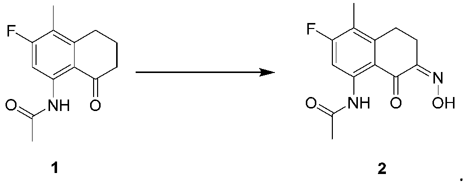

In one embodiment, the preparation method for the compound of formula 2 can comprise the following specific steps:
(a) mixing the base and the solvent, to obtain mixed solution 2;
(b) mixing the mixed solution 2 with the compound of formula 1 to obtain mixed solution 3;
(c) mixing the oximating agent with the mixed solution 3 for carrying out an oximation reaction, to obtain the compound of formula 2;
preferably, the mixing in step (a) being carried out preferably at a temperature of 20-25°C; the mixing in step (b) being carried out preferably at a temperature of 0-5°C; and the mixing in step (c) being carried out preferably at a temperature of 5°C.

In one embodiment, the solvent can be an ether solvent and/or an alcoholic reagent, preferably an ether solvent and an alcoholic reagent. The ether reagent is preferably tetrahydrofuran, and the alcoholic reagent is preferably tert-butanol.

In one embodiment, the base can be an organic base, e.g., potassium tert-butoxide.

In one embodiment, the oximating agent can be one or more of amyl nitrite, isoamyl nitrite, n-butyl nitrite and tert-butyl nitrite, e.g., isoamyl nitrite.

In one embodiment, the molar ratio of the compound of formula 1 to the base can be 1:(2.1-2.3), e.g., 1:2.2.

In one embodiment, the molar ratio of the compound of formula 1 to the oximating agent can be 1:(1.2-1.4), e.g., 1:1.3.

In one embodiment, the mass-to-volume ratio of the compound of formula 1 to the solvent is a conventional mass-to-volume ratio in the art, preferably 90 g/L-100 g/L, e.g., 95.2 g/L.

In one embodiment, the oximation reaction can be carried out at a temperature of 0-5°C, e.g., 5°C.

In one embodiment, the oximation reaction can further comprise a post-processing step, which comprises one or more of quenching, filtration, washing and drying.

The preparation method for the compound of formula 7 can further comprise a preparation method for the compound of formula 6 which can comprise the following step: in a solvent and in the presence of an acid, subjecting a compound of formula 6-2 to a racemization reaction, to obtain the compound of formula 6;

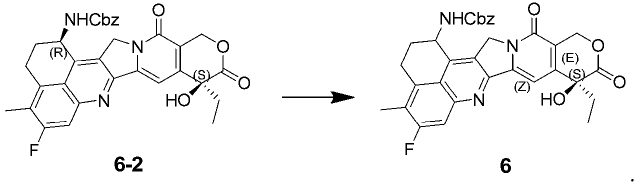

In one embodiment, the solvent can be a halogenated hydrocarbon solvent, e.g., dichloromethane.

In one embodiment, the acid may be an organic acid, e.g., trifluoroacetic acid.

In one embodiment, the molar ratio of the compound of formula 6-2 to the acid can be 1:(3-7), e.g., 1:3.8.

In one embodiment, the mass-to-volume ratio of the compound of formula 6-2 to the solvent can be 50 g/L-200 g/L, e.g., 117.4 g/L.

In one embodiment, the racemization reaction can be carried out at a temperature of 35-45°C, e.g., 40°C.

The preparation method for the compound of formula 7 can further comprise a preparation method for the compound of formula 6-2 which can comprise the following step: in a solvent and in the presence of a base, subjecting a compound of formula 7-1 to a protection reaction with benzyl chloroformate, to obtain the compound of formula 6-2,

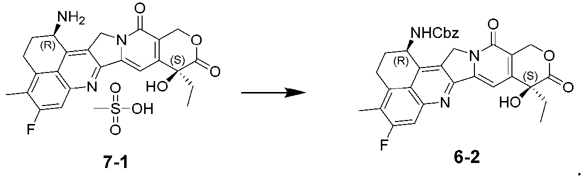

In one embodiment, the compound of formula 7-1 can be a recrystallization mother liquor from the deprotection reaction in the preparation method for the compound of formula 7 as described above.

In one embodiment, the solvent can be a sulfoxide solvent and/or a nitrile solvent, the sulfoxide solvent is preferably dimethyl sulfoxide, and the nitrile solvent is preferably acetonitrile; the solvent is preferably dimethyl sulfoxide and/or acetonitrile, e.g., dimethyl sulfoxide.

In one embodiment, the base can be an organic base, e.g., N,N-diisopropylethylamine.

In one embodiment, the molar ratio of the compound of formula 7-1 to the base can be 1:(3-8), e.g., 1:4.

In one embodiment, the molar ratio of the compound of formula 7-1 to the benzyl chloroformate can be 1:(1.2-2), e.g., 1:1.7.

In one embodiment, the mass-to-volume ratio of the compound of formula 7-1 to the solvent can be 25-33 g/L.

In one embodiment, the protection reaction can be carried out at a temperature of -5-5°C.

In one embodiment, the protection reaction can further comprise a post-processing step, which comprises one or more of crystallization, filtration, washing and drying.

Without departing from the common general knowledge in the art, the above-mentioned preferred conditions can be combined arbitrarily, thereby yielding various preferred examples of the present invention.

The reagents and raw materials used in the present invention are all commercially available.

The positive and advanced effect of the present invention lies in that the preparation method of the present invention uses a small amount of solvent during post-processing, and is easy to filter, and the prepared product is off-white, with higher purity and yield, and is suitable for industrial production. The recrystallization mother liquor obtained from the deprotection reaction is easier to recover, which greatly reduces the cost of raw materials.

### Detailed Description of Embodiments

The present invention will be further illustrated by way of examples below, but the present invention is not limited to the scope of the examples described herein. In the following examples, conventional methods and conditions are used or selected according to the product instructions, unless specific conditions indicated in an experimental method.

### Example 1. Preparation of the compound of formula 2

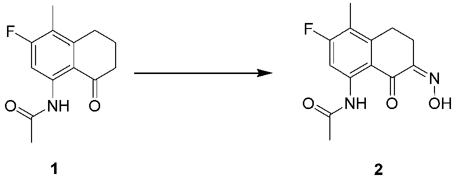

18.9 g of potassium tert-butoxide was added to 170 mL of tetrahydrofuran and 40 mL of tert-butanol at 20-25°C. The mixture was cooled to 0°C, 20 g of compound **1** was added at 0-5°C, and the mixture was reacted under stirring at 0-5°C for 30 minutes. 11.7 g of isoamyl nitrite was added dropwise at 5°C, and the reaction was continued with stirring for 30 min. 240 mL of ice water was added to the reaction solution and the mixture was quenched with 120 mL of 2N dilute hydrochloric acid at 0-10°C. After the mixture was stirred at 10°C for 30 minutes, the product precipitated, followed by filtration and rinsing with 40 mL of methyl tert-butyl ether. After drying, 19.55 g of compound **2** was obtained with a purity of 93.4% and a yield of 80%.

The retention time of compound **2** was 6.333, and the detection conditions are shown in the table below:

| | | | |
|---|---|---|---|
| Instrument model | Thermo U-3000 HPLC, UV detector or other equivalent instruments | | |
| Column model | Waters Xbridge C18, 4.6 × 150 mm, 3.5 µm, PN:186003034 | | |
| Detection wavelength | 220 nm | | |
| Column temperature | 40°C | | |
| Flow rate | 1.0 mL/min | | |
| Injection volume | 5 µL | | |
| Needle washing | Acetonitrile:water = 95:5 (v/v) | | |
| Mobile phase A | A solution of 0.05% trifluoroacetic acid in acetonitrile/water (water:acetonitrile = 95:5), v/v | | |
| Mobile phase B | A solution of 0.05% trifluoroacetic acid in acetonitrile/water (acetonitrile:water = 95:5), v/v | | |

| | Time (min) | A% | B% |
|---|---|---|---|
| | Initial | 100 | 0 |
| Gradient procedure | 8.00 | 0 | 100 |
| | 10.00 | 0 | 100 |
| | 11.00 | 100 | 0 |
| | 15.00 | 100 | 0 |
| Data acquisition time | 15 min | | |

Compound **2:** ¹H NMR (CDCl₃) δ: 11.84 (s, 1H), 8.24 (m, 1H), 2.87 (m, 2H), 2.76 (m, 2H), 2.01 (s, 3H), 1.96 (s, 3H).

### Example 2. Preparation of the compound of formula 3

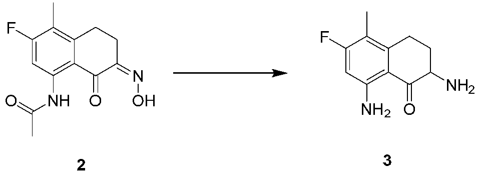

10 g of compound **2** and 0.025 g of Pd/C were added to 7.5 mL of trifluoroacetic acid and 100 mL of methanol. The mixture was purged with nitrogen and hydrogen for 3 times respectively, then reacted for 16 hours under hydrogen at a pressure of 50 psi and at 25°C. 100 mL of methanol was added to dissolve the system, followed by filtration (through celite) and rinsing with 50 mL of methanol, and the resulting filtrate was concentrated to dryness. 100 mL of ethanol was added and the mixture was concentrated to dryness at 55°C. 75 mL of ethanol and 15 mL of 6 N hydrochloric acid were added, and stirred at 65°C for 16 hours. The mixture was concentrated under reduced pressure at 50-70°C to 1V, then 100 mL of ethanol was added and the mixture was concentrated under reduced pressure to 1V. 100 mL of THF solution was added and stirred. The mixture was cooled to 20°C and filtered. After drying, 7.9 g of compound 3 was obtained with a purity of 98.0% and a yield of 78%.

The retention time of compound **3** was 4.918 min, and the detection conditions were the same as those in Example 1.

Compound **3:** ¹H NMR (DMSO) δ: 8.51 (s, 3H), 6.49 (m, 1H), 4.17 (m, 1H), 2.98 (m, 2H), 2.83 (m, 2H), 2.39 (m, 1H), 1.99 (s, 3H).

### Example 3. Preparation of the compound of formula 4

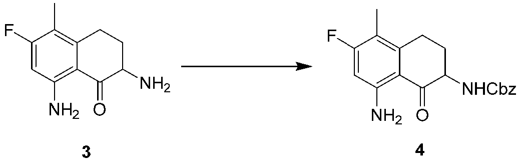

5.7 g of compound **3** and 10.6 g of N,N-diisopropylethylamine were added to 970 mL of tetrahydrofuran, and 5.4 g of benzyl chloroformate was added at -10-0°C. The mixture was then heated to 20°C and reacted for 1 hour. The reaction solution was added to 80 mL of ice water, and the mixture separated into layers. The aqueous phase was extracted twice with 28.5 mL of ethyl acetate each time. The organic phases were combined, washed with 12 mL of brine, dried with sodium sulfate, filtered, and concentrated to dryness. The crude product was slurried with 28.5 mL of methyl tert-butyl ether at 20°C for 2 hours to obtain 4.2 g of compound **4** with a purity of 96.3% and a yield of 69%.

Compound **4:** ¹H NMR (DMSO) δ: 7.39 (m, 7H), 6.38 (m, 1H), 5.07 (s, 1H), 4.24 (m, 1H), 2.92 (m, 1H), 2.83 (m, 1H), 2.13 (m, 1H), 1.98 (s, 3H), 1.91 (m, 1H).

The retention time of compound **4** was 8.381 min, and the detection conditions are shown in the table below:

| | | | |
|---|---|---|---|
| Instrument model | Thermo U-3000 HPLC, UV detector or other equivalent instruments | | |
| Column model | Waters Xbridge C18, 4.6 × 150 mm, 3.5 µm, PN:186003034 | | |
| Detection wavelength | 240 nm | | |
| Column temperature | 40°C | | |
| Flow rate | 1.0 mL/min | | |
| Injection volume | 5 µL | | |
| Needle washing | Acetonitrile:water = 95:5 (v/v) | | |
| Mobile phase A | A solution of 0.05% trifluoroacetic acid in acetonitrile/water (water:acetonitrile = 95:5), v/v | | |
| Mobile phase B | A solution of 0.05% trifluoroacetic acid in acetonitrile/water (acetonitrile:water = 95:5), v/v | | |

| | Time (min) | A% | B% |
|---|---|---|---|
| | Initial | 100 | 0 |
| Gradient procedure | 8.00 | 0 | 100 |
| | 10.00 | 0 | 100 |
| | 11.00 | 100 | 0 |
| | 15.00 | 100 | 0 |
| Data acquisition time | 15 min | | |

### Example 4. Preparation of the compound of formula 5

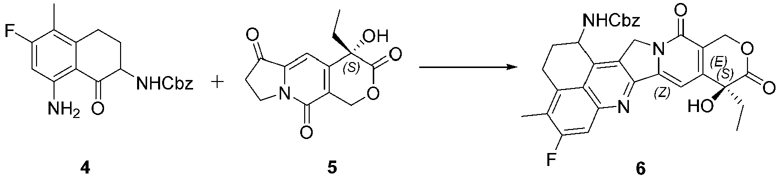

15 g of compound **4,** 12.11 g of compound **5** and 0.754 g of p-toluenesulfonic acid were added to 750 mL of toluene. The mixture was heated to 110°C and reacted for 10 hours. After cooling to 55°C, additional 0.754 g of p-toluenesulfonic acid was added, and the mixture was heated to 110°C and further reacted for 20 hours. After cooling to 15-25°C, the mixture was stirred for 4 hours, followed by filtration and rinsing with 45 mL of toluene. The wet product was slurried with 300 mL of methanol/dichloromethane (equal volume ratio) for 0.5 hours. After the reaction, a small amount of material adhered to the wall. After further slurrying with methanol/dichloromethane, almost no material adhered to the wall. The mixture was filtered, followed by rinsing with 45 mL of methanol/dichloromethane (equal volume ratio) and drying, to obtain 21.38 g of product **6** (the net content: 20.5 g) with a purity of 97.2% and a yield of 82.2%.

The retention times of compound 6 (i.e., a mixture of compounds 6-1 and 6-2) were 8.937 min and 9.080 min. The detection method is shown in the table below:

| | | | |
|---|---|---|---|
| Instrument model | Thermo U-3000 HPLC, UV detector or other equivalent instruments | | |
| Column model | Waters Xbridge C18, 4.6 × 150 mm, 3.5 µm, PN:186003034 | | |
| Detection wavelength | 220 nm | | |
| Column temperature | 40°C | | |
| Flow rate | 1.0 mL/min | | |
| Injection volume | 5 µL | | |
| Needle washing | Acetonitrile:water = 95:5 (v/v) | | |
| Mobile phase A | A solution of 0.05% trifluoroacetic acid in acetonitrile/water (water:acetonitrile = 95:5), v/v | | |
| Mobile phase B | A solution of 0.05% trifluoroacetic acid in acetonitrile/water (acetonitrile:water = 95:5), v/v | | |

| | Time (min) | A% | B% |
|---|---|---|---|
| | Initial | 100 | 0 |
| Gradient procedure | 10.00 | 0 | 100 |
| | 12.00 | 0 | 100 |
| | 13.00 | 100 | 0 |
| | 15.00 | 100 | 0 |
| Data acquisition time | 15 min | | |

Compound **6:** ¹H NMR (DMSO) δ: 8.09 (m, 1H), 7.76 (m, 1H), 7.32 (m, 6H), 5.45 (s, 2H), 5.24 (m, 3H), 5.16 (m, 2H), 3.21 (m, 1H), 3.10 (m, 1H), 2.34 (s, 3H), 2.30 (m, 3H), 1.88 (m, 2H), 0.90 (m, 3H).

### Example 5. Preparation of the compound of formula 7

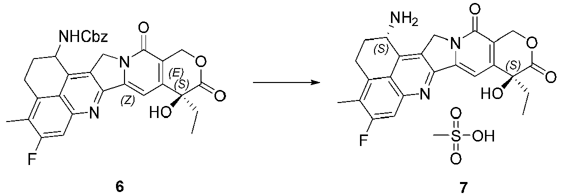

18.7 g of compound **6** was added to 126 g of trifluoroacetic acid, the mixture was stirred at 15-25°C to dissolve, and 27.7 g of methanesulfonic acid was added. The mixture was reacted under stirring at 15-25°C for 1 hour, and 114 mL of ethanol and 171 mL of tetrahydrofuran were slowly added dropwise respectively. After stirring for another 2 hours, the mixture was filtered and the wet product was rinsed with 93.5 mL of tetrahydrofuran. The crude product was dissolved in 250 mL of dimethyl sulfoxide at 60-70°C, and cooled to 50-60°C, and 750 mL of acetonitrile was slowly added dropwise. The mixture was slowly cooled to 15-25°C, and stirring was continued for 10 hours. The mixture was filtered, followed by rinsing with 90 mL of acetonitrile. The crude product was dissolved again in 200 mL of dimethyl sulfoxide at 70-80°C, and cooled to 50-60°C, and 600 mL of acetonitrile was slowly added dropwise. The mixture was slowly cooled to 15-25°C, and stirring was continued for 10 hours. The mixture was filtered, followed by rinsing with 70 mL of acetonitrile. The wet product was slurried with 200 mL of acetonitrile to remove residual dimethyl sulfoxide, followed by filtration and drying, to obtain 7.47 g (net content) of off-white compound **7,** with a purity of 99.9%, an isomer (i.e., compound 7-1) rate of 0.1%, a chiral purity of 99.9%, and a yield of 42.8%.

The retention time of compound **7** was 9.617 min, and the retention time of the isomer (i.e., compound 7-1) was 11.497 min. The detection method is shown in the table below:

| | | | |
|---|---|---|---|
| Instrument model | Agilent 1260 HPLC, UV detector or other equivalent instruments | | |
| Column model | Waters Xbridge C18, 4.6 × 150 mm, 3.5 µm, PN:186003034 | | |
| Detection wavelength | 264 nm | | |
| Column temperature | 45°C | | |
| Sampler tray temperature | 5°C | | |
| Flow rate | 1.2 mL/min | | |
| Injection volume | 5 µL | | |
| Needle washing | Acetonitrile:water = 90:10 (v/v) | | |
| Mobile phase A | A solution of 0.1% trifluoroacetic acid in water | | |
| Mobile phase B | 0.1% trifluoroacetic acid in acetonitrile | | |

| | Time (min) | A% | B% |
|---|---|---|---|
| Gradient procedure | Initial | 90 | 10 |
| | 20.00 | 55 | 45 |
| | 23.00 | 10 | 90 |
| | 28.00 | 10 | 90 |
| | 28.10 | 90 | 10 |
| | 33.00 | 90 | 10 |
| Data acquisition time | 33 min | | |

Compound 7: ¹H NMR (DMSO) δ: 8.49 (s, 3H), 7.92 (m, 1H), 7.38 (s, 1H), 6.60 (s, 1H), 5.77 (m, 1H), 5.49 (m, 3H), 5.14 (s, 1H), 3.32 (s, 1H), 3.15 (m, 1H), 2.58 (m, 2H), 2.46 (s, 3H), 2.34 (s, 3H), 2.25 (m, 1H), 1.92 (m, 1H), 0.92 (m, 1H).
Control: 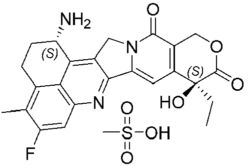
Manufacturer: Changzhou Syntheall Pharmaceuticals Co., Ltd.; lot no.: PC13554-19-SM26-P.

The retention time of compound **7** was 32.76 min which was consistent with that of the control, and the detection method is shown in the table below:

| | | | |
|---|---|---|---|
| Instrument model | Waters Arc HPLC, UV detector or other equivalent instruments | | |
| Column model | Daicel Chiral CEL OJ-H, 4.6×250 mm, 5.0 µm, PN:17325 | | |
| Detection wavelength | 264 nm | | |
| Column temperature | 35°C | | |
| Sampler tray temperature | 25°C | | |
| Flow rate | 1.0 mL/min | | |
| Injection volume | 3 µL | | |
| The solvent for needle washing | Isopropanol | | |
| Mobile phase A | A solution of 0.02% diethanolamine in n-hexane, v/v | | |
| Mobile phase B | A solution of 0.02% diethanolamine in methanol/isopropanol (methanol:isopropanol = 50:50), v/v | | |

| | Time (min) | A% | B% |
|---|---|---|---|
| Gradient procedure | 0.01 | 85 | 15 |
| | 50.00 | 65 | 35 |
| | 60.00 | 50 | 50 |
| | 61.00 | 85 | 15 |
| | 80.00 | 65 | 15 |
| Data acquisition time | 80 min | | |

### Example 6. Preparation of the compound of formula 6-2

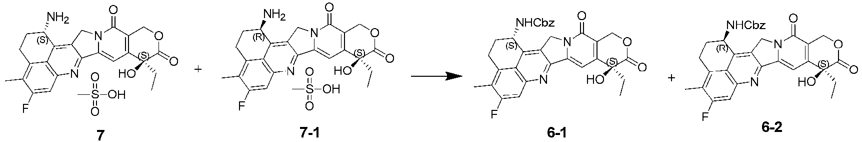

The dimethyl sulfoxide/acetonitrile mother liquor (the net content of the product and isomer: 8.36 g) during the crystallization and purification in Example 5 was concentrated to a volume with no obvious distillate (about 300 mL). After cooling to -5-5°C, 8.2 g of N,N-diisopropylethylamine was added. 4.6 g of benzyl chloroformate was added at a temperature less than 5°C. The mixture was reacted at -5 to -5°C for 1 hour. At a temperature less than 5°C, 300 mL of water was slowly added dropwise and stirring was continued for 1 hour. The mixture was filtered, followed by rinsing with 25 mL of ethanol and drying, to obtain 11.85 g of crude product (a mixture of compounds **6-1** and **6-2**) with a purity of 97.5% (**6-1/6-2**=8.3%/89.2%).

The detection method was the same as in Example 4.

### Example 7. Preparation of the compound of formula 6

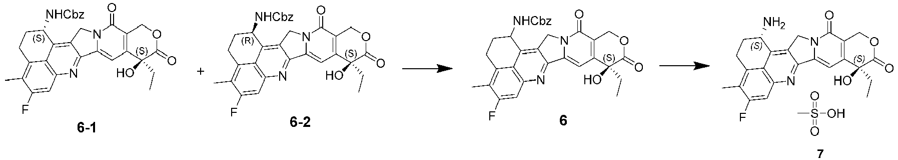

11.85 g of crude product (a mixture of compounds **6-1** and **6-2**) obtained in Example 6 was added to 90 mL of dichloromethane, and 9 g of trifluoroacetic acid was added. The mixture was heated to 40°C and stirred for 15 hours. The dichloromethane in the reaction solution was removed by concentration, the temperature was adjusted to 20°C, and 45 mL of trifluoroacetic acid and 9 mL of methanesulfonic acid were added. The mixture was reacted under stirring at 20°C for 3 hours, 56 mL of ethanol and 84 mL of tetrahydrofuran were slowly added respectively, and the stirring was continued for 2 hours. The mixture was filtered, followed by rinsing with 20 mL of tetrahydrofuran and drying, to obtain 8.8 g of crude product. The crude product was dissolved in 120 mL of dimethyl sulfoxide at 60-70°C, and cooled to 50-60°C, and 360 mL of acetonitrile was slowly added dropwise. The mixture was slowly cooled to 15-25°C, and stirring was continued for 10 hours. The mixture was filtered, followed by rinsing with 30 mL of acetonitrile. The crude product was dissolved again in 90 mL of dimethyl sulfoxide at 70-80°C, and cooled to 50-60°C, and 270 mL of acetonitrile was slowly added dropwise. The mixture was slowly cooled to 15-25°C, and stirring was continued for 10 hours. The mixture was filtered, followed by rinsing with 30 mL of acetonitrile. The wet product was slurried with 300 mL of acetonitrile to remove most of residual dimethyl sulfoxide, followed by filtration and drying, to obtain 3.4 g of compound **7,** with a purity of 99.9%, an isomer rate (i.e., compound 7-1) of 0.07%, a yield of 37.5% (on a corrected-content basis), and an overall recovery yield of 18%.

The detection method was the same as in Example 5.

### Comparative example 1. Preparation of the compound of formula 7

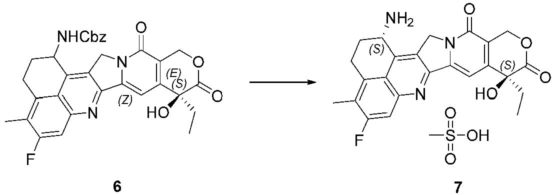

32 g of compound **6** and 6.4 g of Pd/C were added to 1280 mL of N,N-dimethylformamide. The mixture was stirred at 20-30°C under a hydrogen pressure of 15-25 psi for 3-5 h, the reaction solution was filtered, followed by rinsing with 160 mL of N,N-dimethylformamide. The filtrate was diluted with 3840 mL of dichloromethane and 2560 mL of n-heptane. The diluted solution was run through a flash column with 320 g silica gel and eluted with methanol/dichloromethane (1:20, v/v). The column-passed solution was concentrated to 40 V, followed by the addition of 10.8 g of methanesulfonic acid to form a salt. 2560 mL of methyl tert-butyl ether was added to induce crystallization, followed by filtration and rinsing with 160 mL of methyl tert-butyl ether. The crude product was dissolved in 400 mL of dimethyl sulfoxide at 60-70°C, and cooled to 50-60°C, and 1200 mL of acetonitrile was slowly added dropwise. The mixture was slowly cooled to 15-25°C, and stirring was continued for 10 hours. The mixture was filtered, followed by rinsing with 130 mL of acetonitrile. The crude product was dissolved again in 330 mL of dimethyl sulfoxide at 70-80°C, and cooled to 50-60°C, and 990 mL of acetonitrile was slowly added dropwise. The mixture was slowly cooled to 15-25°C, and stirring was continued for 10 hours. The mixture was filtered, followed by rinsing with 110 mL of acetonitrile. The wet product was slurried with 500 mL of acetonitrile to remove residual dimethyl sulfoxide, followed by filtration and drying, to obtain 9.89 g of compound **7,** with a purity of 99.7% and a yield of 32.5% (on a corrected-content basis).

The detection method was the same as in Example 5.

### Comparative example 2. Preparation of the compound of formula 7

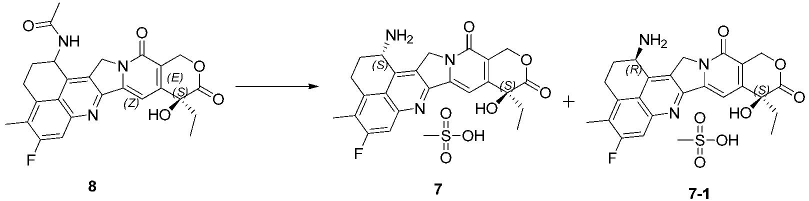

To reproduce step h of Example 3 in CN111470998B, the specific experimental steps were as follows:
8.16 g of compound 8 was added to 163 mL of water, followed by addition of 82 mL of methanesulfonic acid (exothermic reaction). The mixture was heated to 110°C and reacted for 7 h. After cooling to 15-25°C, the black insoluble material in the reaction solution was removed by filtration (6.3 g after drying), followed by rinsing with 25 mL of water. The filtrate was added to the reaction vessel, and 1088 mL of ethanol was slowly added. After stirring for 0.5 h, the mixture was filtered, followed by rinsing with 40 mL of ethanol. The wet product was added to 275 mL of ethanol/water (4:1, v/v), heated to 70-80°C and stirred under reflux for 2 h. After cooling to 15-25°C, the mixture was filtered, followed by rinsing with 32 mL of ethanol and drying, to obtain 2.22 g of compound 7 with a purity of 95.4%, an isomer (i.e., compound 7-1) rate of 3.1%, and a yield of 22.2% (on a corrected-content basis).

## Claims

1. A preparation method for a compound of formula 7, comprising the following step: in an organic solvent, subjecting a compound of formula 6 to a deprotection reaction with methanesulfonic acid, to obtain the compound of formula 7,

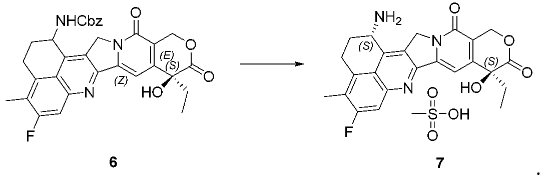

2. The preparation method for a compound of formula 7 of claim 1, wherein the preparation method for the compound of formula 7 satisfies one or more of the following conditions:
(1) the preparation method for the compound of formula 7 comprises the following specific steps: mixing the compound of formula 6 with the organic solvent, then mixing the mixture with the methanesulfonic acid for carrying out a deprotection reaction, to obtain the compound of formula 7; preferably, the mixing is carried out at a temperature of 15-25°C;
(2) the molar ratio of the compound of formula 6 to methanesulfonic acid is 1:(3-15), preferably 1:(3-10), e.g., 1:8.8;
(3) the volume-to-mass ratio of the compound of formula 6 to the organic solvent is 1:(3-10) mL/g, preferably 1:(3-5) mL/g, e.g., 1:4.4 mL/g;
(4) the organic solvent is a haloacetic acid, acetic acid or a chlorinated hydrocarbon solvent, the haloacetic acid is preferably trifluoroacetic acid, the chlorinated hydrocarbon solvent is preferably dichloromethane or chloroform; preferably, the organic solvent is, e.g., trifluoroacetic acid;
(5) the deprotection reaction is carried out at a temperature of 5-40°C, e.g., 15-25°C;
(6) a system of the deprotection reaction contains no water;
(7) the deprotection reaction further comprises a post-processing step, which comprises one or more of crystallization, recrystallization, slurrying, filtration and drying;
(8) a solvent for the crystallization is an alcoholic solvent and/or an ether solvent; the alcoholic solvent is preferably ethanol; the ether solvent is preferably tetrahydrofuran; preferably, the solvent for crystallization is an alcoholic solvent and an ether solvent, e.g., ethanol and tetrahydrofuran; more preferably, the volume ratio of the alcoholic solvent to the ether solvent is 1:(1-2), e.g., 1:1.5;
(9) the mass-to-volume ratio of the compound of formula 6 to the solvent for the crystallization is 65.6 g/L;
(10) a solvent for the recrystallization is a sulfoxide solvent and/or a nitrile solvent, preferably a sulfoxide solvent and a nitrile solvent; the sulfoxide solvent is preferably dimethyl sulfoxide; and the nitrile solvent is preferably acetonitrile;
(11) the volume ratio of the sulfoxide solvent to the nitrile solvent is 1:3;
and (12) a solvent for the slurrying is a nitrile solvent, e.g., acetonitrile.

3. The preparation method for a compound of formula 7 of claim 1 or 2, wherein the preparation method for the compound of formula 7 further comprises a preparation method for the compound of formula 6 comprising the following step: in a solvent and in the presence of a catalyst, carrying out a condensation reaction between a compound of formula 4 and a compound of formula 5, to obtain the compound of formula 6, 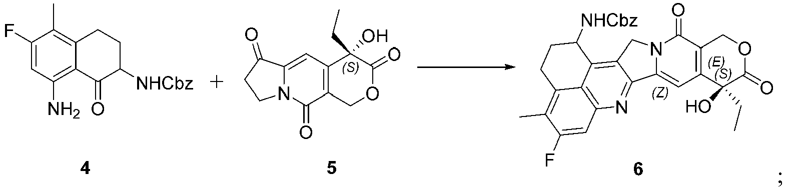
preferably, the preparation method for the compound of formula 6 satisfies one or more of the following conditions:
(1) the preparation method for the compound of formula 6 comprises the following specific steps:
(a) mixing the compound of formula 4, the compound of formula 5, a portion of the catalyst and the solvent for carrying out a condensation reaction, to obtain reaction solution 1;
(b) mixing the remaining catalyst with the reaction solution 1 for carrying out a condensation reaction, to obtain the compound of formula 6;
(2) the solvent is an aromatic solvent, e.g., toluene;
(3) the catalyst is p-toluenesulfonic acid or pyridinium p-toluenesulfonate, e.g., p-toluenesulfonic acid;
(4) the molar ratio of the compound of formula 4 to the compound of formula 5 is 1:(0.9-1.2), e.g., 1:1.05;
(5) the molar ratio of the compound of formula 4 to the catalyst is 1:(0.1-0.3), e.g., 1:0.2;
(6) the mass ratio of the amount of the catalyst added in step (a) to that added in step (b) is 1:1;
(7) the mass-to-volume ratio of the compound of formula 4 to the solvent is 20 g/L-30 g/L, e.g., 20 g/L;
(8) the condensation reaction is carried out at a reflux temperature of the solvent;
(9) the condensation reaction further comprises a post-processing step, which comprises one or more of crystallization, slurrying, filtration and drying;
and (10) the solvent for the slurrying is an alcoholic solvent and/or a halogenated hydrocarbon solvent, preferably an alcoholic solvent and a halogenated hydrocarbon solvent; the alcoholic solvent is preferably methanol; and the halogenated hydrocarbon solvent is preferably dichloromethane.

4. The preparation method for a compound of formula 7 of claim 3, wherein the preparation method for the compound of formula 7 further comprises a preparation method for the compound of formula 4 comprising the following step: in a solvent and in the presence of a catalyst, subjecting a compound of formula 3 to a substitution reaction with benzyl chloroformate, to obtain the compound of formula 4, 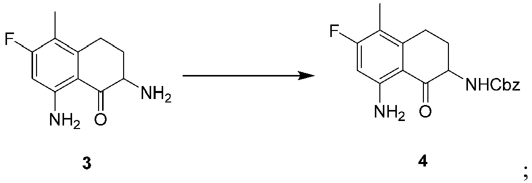
preferably, the preparation method for the compound of formula 4 satisfies one or more of the following conditions:
(1) the preparation method for the compound of formula 4 comprises the following specific steps:
(a) mixing the compound of formula 3, the catalyst and the solvent, to obtain mixed solution 1;
(b) mixing the benzyl chloroformate and the mixed solution 1 for carrying out the substitution reaction, to obtain the compound of formula 4;
preferably, the mixing in step (b) being adding the benzyl chloroformate to the mixed solution 1;
(2) the solvent is an ether solvent, preferably tetrahydrofuran;
(3) the catalyst is N,N-diisopropylethylamine;
(4) the molar ratio of the compound of formula 3 to the catalyst is 1:(3-4), e.g., 1:3;
(5) the molar ratio of the compound of formula 3 to the benzyl chloroformate is 1:(1-1.5), e.g., 1:1.15;
(6) the mass-to-volume ratio of the compound of formula 3 to the solvent is 5 g/L-6 g/L, e.g., 5.9 g/L;
(7) the substitution reaction is carried out at a temperature of 10-20°C, e.g., 20°C;
and (8) the substitution reaction further comprises a post-processing step, which comprises one or more of extraction, washing, filtration, concentration, slurrying and drying.

5. The preparation method for a compound of formula 7 of claim 4, wherein the preparation method for the compound of formula 7 further comprises a preparation method for the compound of formula 3 comprising the following step: in a solvent and in the presence of a catalyst and an acid, carrying out a catalytic hydrogenation reaction of a compound of formula 2 with H₂, to obtain the compound of formula 3, 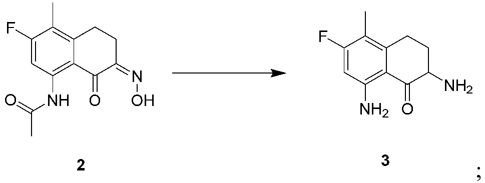
preferably, the preparation method for the compound of formula 3 satisfies one or more of the following conditions:
(1) the preparation method for the compound of formula 3 comprises the following specific steps: mixing the compound of formula 2, the catalyst, the acid and the solvent for carrying out a catalytic hydrogenation reaction under an H₂ atmosphere, to obtain the compound of formula 3;
(2) the solvent is an alcoholic solvent, e.g., methanol;
(3) the catalyst is palladium on carbon (Pd/C), e.g., 10% Pd/C;
(4) the acid is an organic acid, e.g., trifluoroacetic acid;
(5) the mass ratio of the compound of formula 2 to the catalyst is 1:(0.002-0.003), e.g., 1:0.0025;
(6) the molar ratio of the compound of formula 2 to the acid is 1:(2-4), e.g., 1:2.7;
(7) the mass-to-volume ratio of the compound of formula 2 to the solvent is 90 g/L-110 g/L, e.g., 100 g/L;
(8) the catalytic hydrogenation reaction is carried out at a temperature of 20-40°C, e.g., 25°C;
and (9) the catalytic hydrogenation reaction further comprises a post-processing step, which comprises one or more of dissolution, washing, filtration, concentration and drying.

6. The preparation method for a compound of formula 7 of claim 5, wherein the preparation method for the compound of formula 7 further comprises a preparation method for the compound of formula 2 comprising the following step: in a solvent and in the presence of a base, subjecting a compound of formula 1 to an oximation reaction with an oximating agent, to obtain the compound of formula 2, 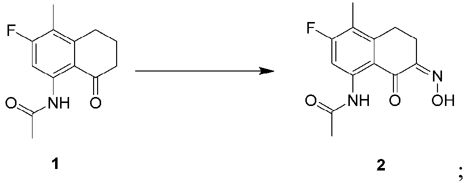 preferably, the preparation method for the compound of formula 2 satisfies one or more of the following conditions:
(1) the preparation method for the compound of formula 2 comprises the following specific steps:
(a) mixing the base and the solvent, to obtain mixed solution 2;
(b) mixing the mixed solution 2 with the compound of formula 1 to obtain mixed solution 3;
(c) mixing the oximating agent with the mixed solution 3 for carrying out an oximation reaction, to obtain the compound of formula 2;
preferably, the mixing in step (a) being carried out preferably at a temperature of 20-25°C; the mixing in step (b) being carried out preferably at a temperature of 0-5°C; and the mixing in step (c) being carried out preferably at a temperature of 5°C;
(2) the solvent is an ether solvent and/or an alcoholic reagent, preferably an ether solvent and an alcoholic reagent; the ether reagent is preferably tetrahydrofuran, and the alcoholic reagent is preferably tert-butanol;
(3) the base is an organic base, e.g., potassium tert-butoxide;
(4) the oximating agent is one or more of amyl nitrite, isoamyl nitrite, n-butyl nitrite and tert-butyl nitrite, e.g., isoamyl nitrite;
(5) the molar ratio of the compound of formula 1 to the base is 1:(2.1-2.3), e.g., 1:2.2;
(6) the molar ratio of the compound of formula 1 to the oximating agent is 1:(1.2-1.4), e.g., 1:1.3;
(7) the mass-to-volume ratio of the compound of formula 1 to the solvent is 90 g/L-100 g/L, e.g., 95.2 g/L;
(8) the oximation reaction is carried out at a temperature of 0-5°C, e.g., 5°C;
and (9) the oximation reaction further comprises a post-processing step, which comprises one or more of quenching, filtration, washing and drying.

7. The preparation method for a compound of formula 7 of claim 1, wherein the preparation method for the compound of formula 7 further comprises a preparation method for the compound of formula 6 comprising the following step: in a solvent and in the presence of an acid, subjecting a compound of formula 6-2 to a racemization reaction, to obtain the compound of formula 6;

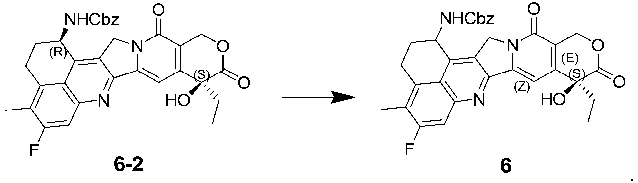

8. The preparation method for a compound of formula 7 of claim 7, wherein the preparation method for the compound of formula 6 satisfies one or more of the following conditions:
(1) the solvent is a halogenated hydrocarbon solvent, e.g., dichloromethane;
(2) the acid is an organic acid, e.g., trifluoroacetic acid;
(3) the molar ratio of the compound of formula 6-2 to the acid is 1:(3-7), e.g., 1:3.8;
(4) the mass-to-volume ratio of the compound of formula 6-2 to the solvent is 50 g/L-200 g/L, e.g., 117.4 g/L;
and (5) the racemization reaction is carried out at a temperature of 35-45°C, e.g., 40°C.

9. The preparation method for a compound of formula 7 of claim 7 or 8, wherein the preparation method for the compound of formula 7 further comprises a preparation method for the compound of formula 6-2 comprising the following step: in a solvent and in the presence of a base, subjecting a compound of formula 7-1 to a protection reaction with benzyl chloroformate, to obtain the compound of formula 6-2,

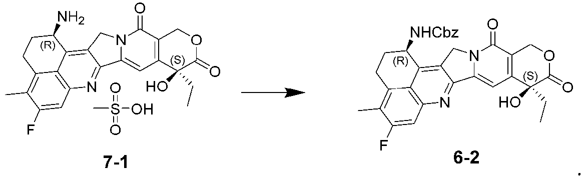

10. The preparation method for a compound of formula 7 of claim 9, wherein the preparation method for the compound of formula 6-2 satisfies one or more of the following conditions:
(1) the compound of formula 7-1 is a recrystallization mother liquor from the deprotection reaction in the preparation method for the compound of formula 7 of any of claims 1-9;
(2) the solvent is a sulfoxide solvent and/or a nitrile solvent, the sulfoxide solvent is preferably dimethyl sulfoxide, and the nitrile solvent is preferably acetonitrile; preferably the solvent is dimethyl sulfoxide and/or acetonitrile, e.g., dimethyl sulfoxide;
(3) the base is an organic base, e.g., N,N-diisopropylethylamine;
(4) the molar ratio of the compound of formula 7-1 to the base is 1:(3-8), e.g., 1:4;
(5) the molar ratio of the compound of formula 7-1 to the benzyl chloroformate is 1:(1.2-2), e.g., 1:1.7;
(6) the mass-to-volume ratio of the compound of formula 7-1 to the solvent is 25-33 g/L;
(7) the protection reaction is carried out at a temperature of -5-5°C;
and (8) the protection reaction further comprises a post-processing step, which comprises one or more of crystallization, filtration, washing and drying.
